# EUROPEAN PATENT SPECIFICATION

(11) **EP 2 737 923 B1**
(45) Date of publication and mention of the grant of the patent: **14.09.2016**
(21) Application number: 12818195.5
(22) Date of filing: 19.07.2012
(51) Int. Cl.: A61K 9/00, A61K 48/00, A61M 5/158, A61M 37/00, A61N 1/04, A61N 1/05, A61N 1/32, B05D 5/00

(54) **Electrode with integrated microneedles for introducing genetic material and a method for its preparation**
Elektrode mit integrierten Mikronadeln zur Einführung von genetischem Material und ein Verfahren zur deren Bereitung
Électrode avec des micro-aiguilles intégrées pour l'introduction de matériau génétique et un procédé pour sa préparation

(30) Priority: 26.07.2011 KR 20110074250
(43) Date of publication of application: 04.06.2014
(73) Proprietor: Raphas Co., Ltd., Seoul 121-912 (KR); Industry-Academic Cooperation Foundation, Yonsei University, Seoul 120-749 (KR)
(72) Inventor: JUNG, Hyungil, Seoul 122-080 (KR); LEE, Kwang, Seoul 139-220 (KR); JEONG, Do Hyeon, Seoul 121-901 (KR); KIM, Jung Dong, Seoul 152-828 (KR)
(74) Representative: Grünecker Patent- und Rechtsanwälte PartG mbB
(86) International application number: PCT/KR2012/005777
(87) International publication number: WO 2013/015563

(56) References cited:
- KR-A- 20070 086 221
- KR-A- 20100 134 237
- US-A1- 2002 082 543
- US-A1- 2009 053 472
- US-A1- 2009 131 905
- US-A1- 2011 177 297
- US-B2- 6 697 669

## Description

### [Technical Field]

The present invention relates to an electro-microneedle integrated body in which a dissolving microstructure and an electrode for electroporation are integrated into one structure for a percutaneous gene delivery in a treatment site, and to a method of preparing the same.

### [Background Art]

Purposes of gene delivery (gene transfer, delivery) and gene therapy are to treat diseases or to develop treatment models by introducing foreign genes to inside of a body to replace a gene or by introducing a required gene to provide a particular function. In particular, attentions were made to cutaneous gene transfer and gene therapy thanks to easy accessibility and monitoring in skin and to the existence of various intracutaneous immunocytes. In the development of delivery technology (gene delivery system) for effective delivery of the foreign gene into a target skin cell and stable expression thereof, the issues to be addressed are the penetration and introduction of a gene into biological walls, that are stratum corneum, the outer-most layer in skin, and cell membranes.

More and more attentions were made to non-viral chemico-physical methods which have a high stability as an adequate delivery system depending on genes and treatment sites, and among them, suggested by Genetronics Inc. is an electroporation method in which intracellular transfection of a gene is conducted with a temporal increase of penetration ratio in cell membrane *in vivo* by an electric field pulse (PCT application, WO99/062592 "Flow-through electroporation system for ex vivo gene therapy"). However, since the electroporation intended for intracellular transfection of a gene needed a sufficient gene concentration in an intracellular electric field pulse site, for this purpose, an additional transdermal delivery means which conventionally uses a disposal injection was required, and as such, an efficient intracutaneous gene delivery to an in-situ treatment site was not available with the said non-integrated system. Accordingly, an integrated system in which transdermal delivery and intracellular gene introduction occur in the in-situ treatment site was proposed, and a focus thereof has been made on an efficient integration between an apparatus for transdermal delivery and electroporation for intracellular gene delivery.

BTX Inc. in the United States suggested a system in which transdermal delivery which uses a disposal injection and intracellular gene delivery method using electroporation were integrated into one apparatus (US granted patent No. 5273525 entitled "Injection and electroporation apparatus for drug and gene delivery"), enabling percutaneous gene delivery in a treatment site. However, the transdermal delivery using a disposal injection caused an intracutaneous stimulus and pain, and further, accurate release of a gene in fine skin membranes was limited. The documents US 2002/082543 A and US 2009/131905 A disclose microneedle devices for transport of molecules across the skin and methods for the preparation of such devices.

Johns Hopkins University introduced an electroporation gene gun in which a gene gun and electroporation were integrated (US patent publication No. 20090030364 entitled "Electroporation gene therapy gun system"), yet the accurate transdermal delivery in the treatment site was also limited with the gene gun. Accordingly, a novel transdermal delivery device integrated with electroporation is required in the art for percutaneous gene delivery in a treatment site, and as an accurate, efficient, and pain-free transdermal delivery device, attentions are paid to a microneedle.

Generally, a microneedle has a diameter and length of micron size, penetrates skin without causing pain, and drug delivery with the microneedle is intended for topical intracutaneous release, not the release in circulatory systems, such as blood vessels or lymph ducts. Talon Biotech suggested a system in which a gene-coated microneedle and an electrode for electroporation were integrated ("Smallpox DNA vaccine delivered by novel skin electroporation device protects mice against intranasal poxvirus challenge," Vaccine 25, 1814-1823, 2007), though the coated microneedle had a limit on an amount of intracutaneous gene release because of a limited amount of gene load, and therefore, the efficiency of percutaneous gene delivery was ultimately low.

Accordingly, required for efficient percutaneous gene delivery in a treatment site is a system in which a dissolving microneedle, which enables more effective gene loading than the coated microneedle, and an electrode for electroporation are integrated. However, it was difficult to prepare a monolithic integrated body in which the dissolving microneedle and the electrode for electroporation are integrated into one structure with a conventional molding method of preparing the dissolving microneedle, that is a micro-casting method.

### [Detailed Description of the Invention]

### [Technical Problem]

The purpose of the present invention is therefore to provide an electro-microneedle integrated body in which a dissolving microneedle and an electrode for electroporation are integrated into one unit, which enables a focused and efficient intracutaneous gene release for percutaneous gene delivery and intracellular gene delivery occurring in an in-situ treatment site and a method of preparing the same.

### [Technical Solution]

In order to accomplish the said purpose, the electro-microneedle integrated body according to the present invention includes an electrode for electroporation suitable for contacting the skin of a human body to apply an electric field pulse, the electrode including a base part and a plurality of electrode parts protruding from the base part, and a microneedle adhered to each electrode part and insertable into the skin of a human body, each microneedle including a biocompatible and biodegradable viscous material and a genetic material, wherein the microneedle can degrade within the skin, and wherein an electric field pulse can be applied through the electrode for electroporation in a site in which the microneedle is inserted.

A method of preparing an electro-microneedle integrated body according to the present invention includes in a first step, preparing an electrode for electroporation including a base part and a plurality of electrode parts protruding from the base part, in a second step, preparing a mixed composition by mixing a biocompatible and biodegradable viscous material and a genetic material and forming a microneedle in a this step, array by supplying the mixed composition in a liquid state on the electrode parts and coagulate it.

According to an embodiment of the present invention, the forming of the microneedle may include applying the mixed composition in a liquid state on a plate-like substrate, contacting the electrode parts with the applied mixed composition, drawing the mixed composition by making a relative movement of the substrate over the electrode parts and coagulating the drawn mixed composition.

According to another embodiment of the present invention, the method may further include, after coagulating the drawn mixed composition, liquefying the mixed composition on a side of the substrate by heating the substrate.

According to the present invention, the genetic materials can be delivered intensively in the treatment site, and efficiency of intracellular gene delivery can be increased by an application of an electric field pulse in the same site.

### [Brief Description of the Drawings]

FIG. 1 illustrates a flowchart showing a method of preparing an electro-microneedle integrated body according to one embodiment of the present invention.
FIG. 2 and FIG. 3 illustrate rough perspective views of an electrode for electroporation.
FIG. 4 and FIG. 5 illustrate rough flowcharts showing a method of forming a microneedle.

### [Best Mode of the Invention]

An electro-microneedle integrated body and a method of preparing the same according to embodiments of the present invention will be described in detail below with reference to the accompanying drawings.

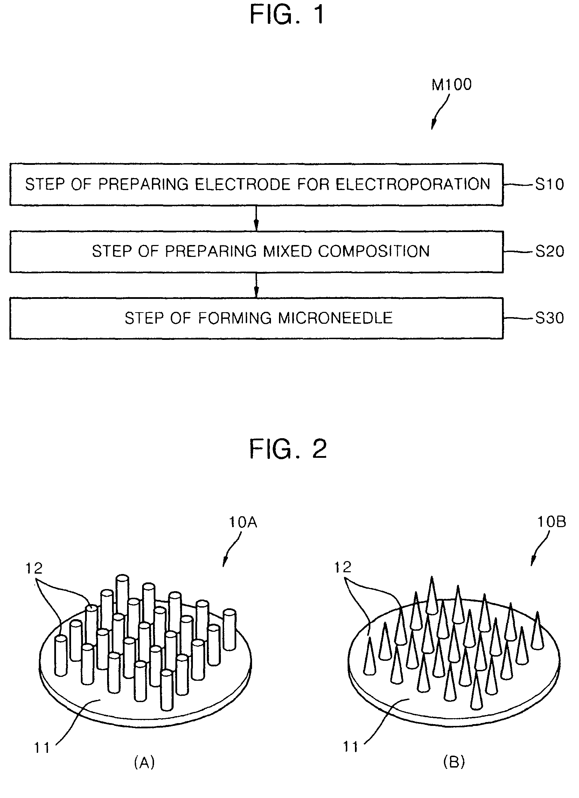

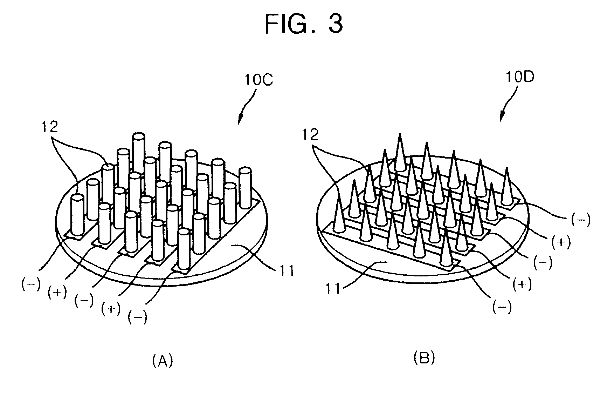

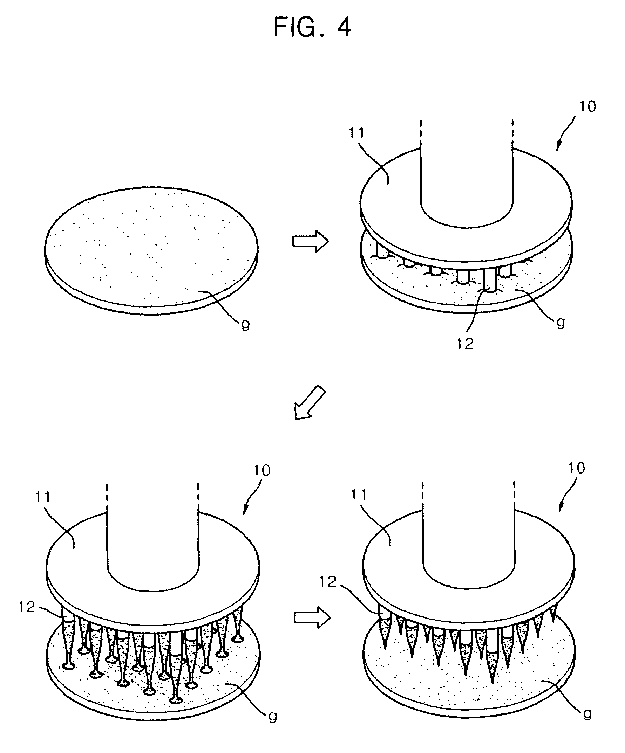

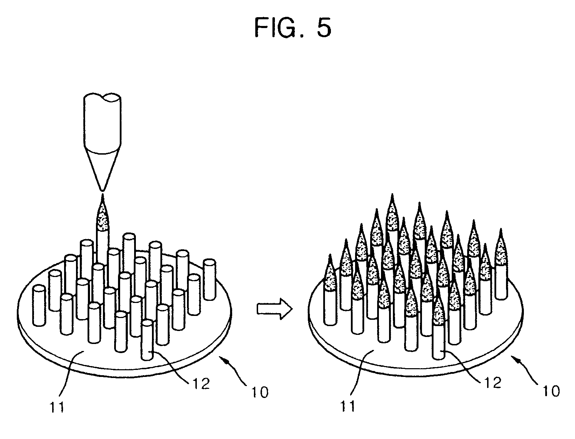

FIG. 1 illustrates a flowchart showing a method of preparing an electro-microneedle integrated body according to one embodiment of the present invention, FIG. 2 and FIG. 3 illustrate rough perspective views of an electrode for electroporation, and FIG. 4 and FIG. 5 illustrate rough flowcharts showing a method of forming a microneedle.

Referring to FIG. 1 to FIG. 5, the method of preparing an electro-microneedle integrated body according to the present embodiment (M100) includes preparing an electrode for electroporation (S10), preparing a mixed composition (S20), and forming a microneedle (S30).

The preparing of the electrode for electroporation (S10) is a step to prepare an electrode for electroporation. The electrode for electroporation (10A to 10D) is intended for electroporation, i.e. for an application of an electric field pulse so that a genetic material (gene) delivered within skin by a microneedle penetrates to cells smoothly. As illustrated in FIG. 2 and FIG. 3, the electrode for electroporation (10A to 10D) possesses a plate-like base part 11, and a plurality of electrode parts 12 protruding from the base part 11. Herein, the shape of the electrode parts 12 can be various as illustrated in FIG. 2 and FIG. 3. For instance, the electrode parts can be micro-blades, micro-knives, micro-fibers, micro-spikes, micro-probes, or micro-barbs, more preferably, micro-spikes, micro-probes, or micro-barbs, and most preferably micro-spikes or micro-probes.

The electrode for electroporation can be prepared using various micro-fabrication technologies and metal deposition technologies either alone or in combination thereof. Herein, the micro-fabrication technologies denote technologies for cutting and abrasion of a metal and polymer, and the metal deposition technologies denote technologies for selective metal deposition and metal plating on a fabricated polymer.

As illustrated in FIG. 2, the electrode for electroporation (10A and 10B) can be prepared in a form of a metal-integrated microstructure in which the base part and the electrode parts are integrated into one body using a metal. Herein, the metal is preferably a metal which can be applied in a living body, and can be formed with ceramic or semiconductor. In particular, materials for the electrode for electroporation are cobalt, titanium, stainless steel, chromium, nickel, copper, silver, gold, aluminum, or alloys thereof, more preferably cobalt, titanium, stainless steel, chromium, nickel, copper, aluminum, or alloys thereof, and most preferably titanium, chromium, nickel, aluminum, or alloys thereof.

The metal-integrated electrode for electroporation can be prepared with various metal micro-fabrication technologies known in the art. For instance, the metal micro-fabrication can be conducted using rolling, forging, milling, cutting, or turning processing. Further, an effective bottom diameter of the electrode parts of the electrode for electroporation is 10 to 1000 µm, and more preferably 50 to 500 µm. An effective aspect ratio (length:bottom diameter) is 0.1 to 10, and more preferably 0.5 to 5.

Meanwhile, as illustrated in FIG. 3, the electrode for electroporation (10C and 10D) can be prepared in a metal-patterned polymer form. That is, the patterned form can be prepared by first preparing a basic structure having the base part and electrode parts using a polymer and by a subsequent metal deposition or metal plating on an entire structure, or selective metal deposition or metal plating on the electrode parts.

Herein, the polymer is preferably biocompatible and can be, in particular, polymers such as acrylic acid, acrylic acid ester, acryl amide, acrylonitrile, methacrylic acid, methacrylic acid ester and copolymers thereof, and more preferably methacrylic resin or polyacrylic acid resin. The metal deposition can be performed by various methods known in the art, physical metal deposition or chemical metal deposition can be preferably used. and most preferably, the metal can be deposited using physical metal deposition of sputtering or evaporation or chemical metal deposition of Tollens' reaction. In addition, metal plating can be further included after the metal deposition. The metal used in deposition or plating is one that can be applicable in a living body, preferably cobalt, titanium, stainless steel, chromium, nickel, copper, silver, gold, aluminum, or alloys thereof, more preferably cobalt, titanium, stainless steel, chromium, nickel, copper, aluminum, or alloys thereof, and most preferably titanium, chromium, nickel, aluminum or alloys thereof.

Meanwhile, the metal-integrated electrode for electroporation illustrated in FIG. 2 can be only used as one electrode, and the metal-patterned electrode for electroporation illustrated in FIG 3 can be used as both electrodes (cathode and anode).

In the preparing of the mixed composition (S20), the mixed composition is prepared by mixing a viscous material and a genetic material. Herein, the viscous material is biocompatible, biodegradable and a material dissolvable *in vivo.* The "term biocompatible material" denotes a material which does not have any substantial toxicity in a human body, which is chemically inert and which does not have immunogenicity, the "term biodegradable material" denotes a material which can be decomposed by body fluids or microorganisms in a living body, and the "term material dissolvable *in vivo*" denotes a material which can be dissolved by temperature or body fluids in a living body.

The viscous material used in the present invention is carbohydrate, more preferably monosaccharide, disaccharide, trisaccharide, oligosaccharide, polysaccharide or alcohol derivatives thereof. More preferably, the viscous material includes glucose, lactose, fructose, galactose, mannose, malturose, lacturose, maltose, sucrose, trehalose, raffinose, melezitose, melibiose, xylobiose, cellobiose, stachyose, sorbitol, mannitol, erythritol, xylitol, lacitol, maltitol; aldonic acids and lactone derivatives thereof, such as gluconic acid and gluconic acid γ-lactone, aldaric acids and lactone derivatives thereof, such as ribaraic acid, arabinaric acid, and galactaric acid; uronic acids, such as glucuronic acid, galaccuronic, acid and mannuronic acid; starch, vegetable gums, substituted cellulose (e.g., carboxymethylcellulose, hydroxyethylcellulose, and alkylcellulose), crystalline cellulose, heparin, hyaluronic acid, chitosan, dextran, alginate, tragacanth, agar and carrageenan.

Furthermore, the viscous material used in the present invention has a viscosity in a liquid state. Such a viscosity can be varied depending on type, concentration, air condition and temperature, and can be adjusted in accordance with the purpose of the present invention. Preferably, the viscous material used in the present invention shows a viscosity of no more than 0.2 m²/s (200000 cSt).

Meanwhile, liquefying of the viscous material can be conducted by various methods known in the art. According to an embodiment of the present invention, the liquefying can be performed by heating at a temperature of at least a melting point of the biocompatible viscous material or by dissolving the biocompatible material in an appropriate solvent (for example, water, water-free or water-containing lower alcohol with 1 to 4 carbon atoms, acetone, ethyl acetate, chloroform, 1,3-butylene glycol, hexane, diethyl ether, butylacetate).

According to the present embodiment, the viscous material has a melting point of at least 50°C, more preferably at least 60°C, still more preferably at least 70°C, and most preferably at least 80°C. When describing the melting point, an upper limit is not particularly limited and can be preferably 500°C, more preferably 400°C, still more preferably 300°C, and most preferably 200°C.

The genetic material (gene) is intended for gene therapy and is a nucleic acid molecule, such as DNA, RNA, siRNA, and microRNA for antiinflammatory drug, antiarthritic drug, anticonvulsive drug, antidepressant drug, antipsychotic drug, tranquilizer, antianxiety drug, narcotic antagonist, antiparkinsonism drug, cholinergic agonist, anticancer drug, antiangiogenic drug, immunosuppressive drug, antiviral drug, antibiotic, orexigenic drug, anticholinergic drug, antihistamine drug, antimigraine drug, hormone drug, vasodilator for coronary artery, cerebrovascular, or peripheral blood vessel, contraceptive, antithrombotic drug, diuretic drug, antihypertensive drug, and drug for cardiovascular disease. Preferably, the genetic material includes hormone, hormone agonist, enzyme, enzyme inhibitor, signal delivery protein or portion thereof, antibody or portion thereof, single chain antibody, binding protein or binding domain thereof, antigen, adhesion protein, structural protein, regulatory protein, toxoprotein, cytokine, transcriptional regulatory factor, blood coagulation factor and vaccine.

More particularly, the genetic material related to protein/peptide/vaccine includes insulin, insulin-like growth factor 1 (1GF-1), growth hormone, erythropoietin, granulocyte-colony stimulating factors (G-CSFs), granulocyte/macrophage-colony stimulating factors (GM-CSFs), interferon alpha, interferon beta, interferon gamma, interleukin-1 alpha and beta, interleukin-3, interleukin-4, interleukin-6, interleukin-2, epidermal growth factors (EGFs), calcitonin, adrenocorticotropic hormone (ACTH), tumor necrosis factor (TNF), atobisban, buserelin, cetrorelix, deslorelin, desmopressin, dynorphin A (1-13), elcatonin, eleidosin, eptifibatide, growth hormone releasing hormone-II (GHRH-II), gonadorelin, goserelin, histrelin, leuprorelin, lypressin, octreotide, oxytocin, pitressin, secretin, sincalide, terlipressin, thymopentin, thymosin αl, triptorelin, bivalirudin, carbetocin, cyclosporine, exedine, lanreotide, luteinizing hormone-releasing hormone (LHRH), nafarelin, parathyroid gland hormone, pramlintide, enfuvirtide (T-20), thymalfasin, ziconotide, antivirus and vaccine protein.

A mixing of the genetic material and the viscous material can be performed using various methods known in the art. A basic mixing may be available through a stirrer, and the preparation can be conducted in oil-in-water or water-in-oil emulsion type and multiple-emulsion type. A method for preparing an emulsion can be performed by dispersing the genetic material directly in the viscous material to prepare the emulsion in which the genetic material is included therein, preferably in the absence of a emulsifier, or using various natural or synthetic emulsifiers.

When the emulsifier is used, the emulsion in which the gene is included therein is more preferably stabilized using natural emulsifiers, such as lecithin, borax, stearic acid, amizole soft, helio gel, beeswax, xanthan gum, emulsifying wax, and solubilizer, or is prepared using at least one selected from group consisting of synthetic emulsifiers, including emulsifiers for oil-in-water, such as PEG-8 dilaurate, PEG-150 distearate, PEG-8 stearate, PEG-40 distearate, PEG-100 distearate, or emulsifiers for water-in-oil emulsion, such as sorbitan stearate, sorbitan oleate, sorbitan sesquioleate, sorbitan trioleate, and combinations thereof. Most preferably, the emulsion is prepared without emulsifier. For instance, the mixed composition can be prepared by emulsifying a lipid-soluble liquid containing the genetic material in an aqueous viscous material in an oil-in-water (W/O) type with a homogenizer or by emulsifying an aqueous liquid containing the genetic material in a lipid-soluble viscous material in a water-in-oil (O/W) type with a homogenizer.

The forming of the microneedle (S30) is performed by forming a microneedle on the electrode parts 12 of the electrode for electroporation using the mixed composition, and in particular, is performed as follows.

As illustrated in FIG. 4, the mixed composition is applied on a plate-like substrate g, and then, the electrode parts of the electrode for electroporation are contacted with the applied mixed composition. Thereafter, a relative movement of an electrode for electroporation 10 is performed over the substrate g to draw the mixed composition, and when time passes, both the drawn mixed composition and remaining mixed composition on the substrate coagulate. When the substrate is heated in this state, the viscous composition on the substrate is liquefied and thus the drawn viscous composition and the viscous composition on the substrate are separated, thereby forming the microneedle including the mixed composition on each electrode part.

Meanwhile, the microneedle may be formed by spotting the mixed composition in each electrode part 12, and then, coagulating the same, as illustrated in FIG 5.

Furthermore, the electrode parts of the electrode for electroporation are contacted with the mixed composition in a state in which the mixed composition is applied on a plate-like substrate. Thereafter, when the mixed composition is drawn in a state maintaining at least a certain temperature of the substrate so that the mixed composition on the substrate does not coagulate, the viscous composition on a side of the electrode for electroporation coagulates quickly while the viscous composition on a side of the substrate coagulates relatively slowly, and thus, it is drawn in a structure of which diameter decreases as being closer to the substrate. When the drawing is continuously performed in this state, the coagulated viscous composition is separated from the substrate, thereby forming the microneedle in each electrode part.

Meanwhile, the thusly-formed microneedle has a top diameter of 1 to 100 µm, more preferably 2 to 50 µm, and most preferably 5 to 20 µm.

In addition, an effective length of the microneedle is not particularly limited, and the length can be varied depending on the type of genes to be delivered, a gene delivery site and a position for conducting electroporation. According to the present embodiment, the effective length of the microneedle is preferably 100 to 10,000 µm, more preferably 200 to 10,000 µm, still more preferably 300 to 8,000 µm, and most preferably 500 to 2,000 µm.

Herein the term, "top" of the microneedle denotes an end part of the microneedle having a minimum diameter. Further, the term "effective length of microneedle" denotes a vertical length from the top of the microneedle to the upper surface of the electrode parts.

The microneedle and the electrode parts are to be inserted into skin, whereby the microneedle win degrade within the skin, thereby releasing the genetic material (gene) contained therein. When power is applied to the electrode for electroporation in this state, an electric field pulse is applied in a region in which the genetic material is released, thereby enabling a smooth delivery of the genetic material into cells.

As explained above, with the present integrated body, a genetic material can be intensively delivered in a treatment site, an electric field pulse can be applied in the same site, and the efficiency of intracellular gene delivery can be increased. The present invention is defined by the appended claims.

## Claims

1. An electro-microneedle integrated body, comprising:
an electrode for electroporation which is to be contacted with skin of a human body to apply an electric field pulse, including a base part and a plurality of electrode parts protruding from the base part; and a set of microneedles, one
microneedle adhered to each electrode part and insertable into the skin of a human body each microneedle, comprising a biocompatible and biodegradable material and a genetic material,
wherein each microneedle is to degrade within the skin, and an electric field pulse is to be applied through the electrode for electroporation in a site in which the microneedles are to be inserted to enable a smooth introduction of the genetic material contained in the microneedles into cells,
whereby the microneedles are preparable by mixing a biocompatible and biodegradable viscous material and the genetic material into a mixed composition **characterized in that** the microneedles are formable by supplying the mixed composition in a liquid state onto each of the plurality of electrode parts the documents US 2002/082543 A and US 2009/131905 A disclose microneedle devices 2 for transport of molecules across the skin and methods for the preparation of such devices **characterized in that** protruding from the base part and congulating the mixed composition.

2. The electro-microneedle integrated body according to claim 1, wherein the biocompatible and biodegradable viscous material is selected from the group consisting of polyester, polyhydroxyalkanoate (PHAs), polyα-hydroxyacid, polyβ-hydroxyacid, poly3-hydroxybutyrate-co-valerate; (PHBV), poly3-hydroxyproprionate; (PHP), poly3-hydroxyhexanoate; (PHH), poly4-hydroxyacid, poly4-hydroxybutyrate, poly4-hydroxyvalerate, poly4-hydroxyhexanoate, polyester amide, polycaprolactone, polylactide (PLA), polyglycolide (PGA), polylactide-co-glycolide;(PLGA), polydioxanone, polyorthoester, polyanhydride, polyglycolic acid-co-trimethylene carbonate, polyphosphoester, polyphosphoester urethane, polylamino acid, polycyanoacrylate, polytrimethylene carbonate, polyiminocarbonate, polytyrosine carbonate, polycarbonate, polytyrosine acrylate, polyalkylene oxalate, polyphosphagens, PHA-PEG, polyvinylpyrrolidone, polybutadiene, polyhydroxybutyric acid, polymethyl methacrylate, polymethacrylic acid ester, polypropylene, polystyrene, polyvinyl acetal diethylamino acetate, polyvinyl acetate, polyvinyl alcohol, polyvinyl butyral, polyvinyl formal, vinylchloride-propylene-vinylacetate copolymer, vinylchloride-vinylacetate copolymer, cumaroneindene polymer, dibutylaminohydroxypropyl ether, ethylene-vinylacetate copolymer, glycerol distearate, 2-methyl-5-vinylpyridine methacrylate-methacrylic acid copolymer, hyaluronic acid, myristic acid, palmitic acid, stearic acid, behenic acid, cellulose or derivatives thereof, maltose, dextran, glucomannan, glucosamine, chitosan, heparin, alginate, inulin, starch, glycogen, chitin, chondroitin, dextrin, keratan sulfate, beef tallow, whale wax, beeswax, paraffin wax, and castor wax.

3. A method of preparing an electro-microneedle integrated body, comprising:
in a first step, preparing an electrode for electroporation including a base part and a plurality of electrode parts protruding from the base part;
in a second step, preparing a mixed composition by mixing a biocompatible and biodegradable viscous material and a genetic material; and
in a third step, forming a microneedle on each electrode pout by supplying the mixed composition in a liquid state onto each of the electrode parts and coagulate the mixed composition.

4. The method according to claim 3, wherein the forming of the microneedle comprises:
applying the mixed composition in a liquid state on a plate-like substrate;
contacting the electrode parts with the applied mixed composition;
drawing the mixed composition by making a relative movement of the electrode parts over the substrate; and
coagulating the drawn mixed composition.

5. The method according to claim 4, further comprising, after coagulating the drawn mixed composition, separating the coagulated mixed composition from the substrate by liquefying the mixed composition on a side of the substrate by heating the substrate.

6. The method according to claim 3, wherein the forming of the microneedle comprises:
spotting the mixed composition in a liquid state on the electrode parts; and
coagulating the spotted mixed composition.

7. The method according to claim 3, wherein the biocompatible and biodegradable viscous material is selected from the group consisting of polyester, polyhydroxyalkanoate (PHAs), polyα-hydroxyacid, polyβ-hydroxyacid, poly3-hydroxybutyrate-co-valerate; (PHBV), polyl 3-hydroxyproprionate; (PHP), poly3-hydroxyhexanoate; (PHH), poly4-hydroxyacid, poly4-hydroxybutyrate, poly4-hydroxyvalerate, poly4-hydroxyhexanoate, polyester amide, polycaprolactone, polylactide (PLA), polyglycolide (PGA), polylactide-co-glycolide; (PLGA), polydioxanone, polyorthoester, polyanhydride, polyglycolic acid-co-trimethylene carbonate, polyphosphoester, polyphosphoester urethane, polyamino acid, polycyanoacrylate, polytrimethylene carbonate, polyiminocarbonate, polytyrosine carbonate, polycarbonate, polytyrosine arylate, polyalkylene oxalate, polyphosphagens, PHA-PEG, polyvinylpyrrolidone, polybutadiene, polyhydroxybutyric acid, polymethyl methacrylate, polymethacrylic acid ester, polypropylene, polystyrene, polyvinyl acetal diethylamino acetate, polyvinyl acetate, polyvinyl alcohol, polyvinyl butyral, polyvinyl formal, vinylchloride-propylene-vinylacetate copolymer, vinylchloride-vinylacetate copolymer, cumaroneindene polymer, dibutylaminohydroxypropyl ether, ethylene-vinylacetate copolymer, glycerol distearate, 2-methyl-5-vinylpyridine methacrylate-methacrylic acid copolymer, hyaluronic acid, myristic acid, palmitic acid, stearic acid, behenic acid, cellulose or derivatives thereof, maltose, dextran, glucomannan, glucosamine, chitosan, heparin, alginate, inulin, starch, glycogen, chitin, chondroitin, dextrin, keratan sulfate, beef tallow, whale wax, beeswax, paraffin wax and castor wax.

## Patentansprüche

1. Integrierter Elektro-Mikronadelkörper, umfassend:
eine Elektrode zur Elektroporation, welche mit der Haut eines menschlichen Körpers in Kontakt gebracht werden soll, um einen elektrischen Feldpuls anzulegen, die ein Basisteil und eine Mehrzahl von Elektrodenteilen einschließt, die aus dem Basisteil hervorstehen; und
einen Satz von Mikronadeln, wobei eine Mikronadel an jedem Elektrodenteil anhaftet und in die Haut eines menschlichen Körpers einführbar ist, wobei jede Mikronadel ein bio-kompatibles und biologisch abbaubares Material und ein genetisches Material umfasst,
wobei jede Mikronadel in der Haut abgebaut werden soll, und ein elektrischer Feldpuls durch die Elektrode zur Elektroporation an einer Stelle angelegt werden soll, an der die Mikronadeln eingeführt werden sollen, um eine reibungslose Einführung des in den Mikronadeln enthaltenen genetischen Materials in Zellen zu ermöglichen,
wobei die Mikronadeln durch Vermischen eines biokompatiblen und biologisch abbaubaren viskosen Materials und des genetischen Materials zu einer gemischten Zusammensetzung hergestellt werden können,
**dadurch gekennzeichnet, dass** die Mikronadeln durch Zuführen der gemischten Zusammensetzung in einem flüssigen Zustand zu jedem von der Mehrzahl von Elektrodenteilen, die aus dem Basisteil hervorstehen, und Koagulieren der gemischten Zusammensetzung gebildet werden können.

2. Integrierter Elektro-Mikronadelkörper gemäß Anspruch 1, wobei das biokompatible und biologisch abbaubare viskose Material ausgewählt ist aus der Gruppe bestehend aus Polyester, Polyhydroxyalkanoat (PHAs), Poly-α-hydroxysäure, Poly-β-hydroxysäure, Poly-3-hydroxybutyrat-co-valerat; (PHBV), Poly-3-hydroxyproprionat; (PHP), Poly-3-hydroxyhexanoat; (PHH), Poly-4-hydroxysäure, Poly-4-hydroxybutyrat, Poly-4-hydroxyvalerat, Poly-4-hydroxyhexanoat, Polyesteramid, Polycaprolacton, Polylactid (PLA), Polyglycolid (PGA), Polylactid-co-glycolid; (PLGA), Polydioxanon, Polyorthoester, Polyanhydrid, Polyglycolsäure-co-trimethylencarbonat, Polyphosphoester, Polyphosphoester-urethan, Polyaminosäure, Polycyanoacrylat, Polytrimethylencarbonat, Polyiminocarbonat, Polytyrosincarbonat, Polycarbonat, Polytyrosinarylat, Polyalkylen-oxalat, Polyphosphagenen, PHA-PEG, Polyvinylpyrrolidon, Polybutadien, Polyhydroxybuttersäure, Polymethylmethacrylat, Polymethacrylsäureester, Polypropylen, Polystyrol, Polyvinylacetal-diethylamino-acetat, Polyvinylacetat, Polyvinylalkohol, Polyvinylbutyral, Polyvinylformal, Vinylchlorid-Propylen-Vinylacetat-Copolymer, Vinylchlorid-Vinylacetat-Copolymer, Cumaronindenpolymer, Dibutylaminohydroxypropylether, Ethylen-Vinylacetat-Copolymer, Glyceroldistearat, 2-Methyl-5-vinylpyridin-methacrylat-Methacrylsäure-Copolymer, Hyaluronsäure, Myristinsäure, Palmitinsäure, Stearinsäure, Behensäure, Cellulose oder Derivate davon, Maltose, Dextran, Glucomannan, Glucosamin, Chitosan, Heparin, Alginat, Inulin, Stärke, Glycogen, Chitin, Chondroitin, Dextrin, Keratansulfat, Rindertalg, Walwachs, Bienenwachs, Paraffinwachs und Castorwachs.

3. Verfahren zum Herstellen eines integrierten Elektro-Mikronadelkörpers, umfassend:
in einem ersten Schritt, das Herstellen einer Elektrode zur Elektroporation, die ein Basisteil und eine Mehrzahl von Elektrodenteilen einschließt, die aus dem Basisteil hervorstehen;
in einem zweiten Schritt, das Herstellen einer gemischten Zusammensetzung durch Vermischen eines biokompatiblen und biologischen abbaubaren viskosen Materials und eines genetischen Materials; und
in einem dritten Schritt, das Bilden einer Mikronadel auf jedem Elektrodenteil durch Zuführen der gemischten Zusammensetzung in einem flüssigen Zustand zu jedem der Elektrodenteile und das Koagulieren der gemischten Zusammensetzung.

4. Verfahren gemäß Anspruch 3, wobei das Bilden der Mikronadel umfasst:
das Aufbringen der gemischten Zusammensetzung in einem flüssigen Zustand auf ein plattenartiges Substrat;
das Inkontaktbringen der Elektrodenteile mit der aufgebrachten gemischten Zusammensetzung;
das Ziehen der gemischten Zusammensetzung durch Ausführen einer relativen Bewegung der Elektrodenteile über dem Substrat;
das Koagulieren der gezogenen gemischten Zusammensetzung.

5. Verfahren gemäß Anspruch 4, außerdem umfassend, nach dem Koagulieren der gezogenen gemischten Zusammensetzung, das Trennen der koagulierten gemischten Zusammensetzung von dem Substrat durch Verflüssigen der gemischten Zusammensetzung auf einer Seite des Substrats durch Erwärmen des Substrats.

6. Verfahren gemäß Anspruch 3, wobei das Bilden der Mikronadel umfasst:
das Auftupfen der gemischten Zusammensetzung in einem flüssigen Zustand auf die Elektrodenteile; und
das Koagulieren der aufgetupften gemischten Zusammensetzung.

7. Verfahren gemäß Anspruch 3, wobei das biokompatible und biologisch abbaubare viskose Material ausgewählt ist aus der Gruppe bestehend aus Polyester, Polyhydroxyalkanoat (PHAs), Poly-α-hydroxysäure, Poly-β-hydroxysäure, Poly-3-hydroxybutyrat-co-valerat; (PHBV), Poly-3-hydroxyproprionat; (PHP), Poly-3-hydroxyhexanoat; (PHH), Poly-4-hydroxysäure, Poly-4-hydroxybutyrat, Poly-4-hydroxyvalerat, Poly-4-hydroxyhexanoat, Polyesteramid, Polycaprolacton, Polylactid (PLA), Polyglycolid (PGA), Polylactid-co-glycolid; (PLGA), Polydioxanon, Polyorthoester, Polyanhydrid, Polyglycolsäure-co-trimethylencarbonat, Polyphosphoester, Polyphosphoester-urethan, Polyaminosäure, Polycyanoacrylat, Polytrimethylencarbonat, Polyiminocarbonat, Polytyrosincarbonat, Polycarbonat, Polytyrosinarylat, Polyalkylenoxalat, Polyphosphagenen, PHA-PEG, Polyvinylpyrrolidon, Polybutadien, Polyhydroxybuttersäure, Polymethylmethacrylat, Polymethacrylsäureester, Polypropylen, Polystyrol, Polyvinylacetal-diethylamino-acetat, Polyvinylacetat, Polyvinylalkohol, Polyvinylbutyral, Polyvinylformal, Vinylchlorid-Propylen-Vinylacetat-Copolymer, Vinylchlorid-Vinylacetat-Copolymer, Cumaronindenpolymer, Dibutylaminohydroxypropylether, Ethylen-Vinylacetat-Copolymer, Glyceroldistearat, 2-Methyl-5-vinylpyridin-methacrylat-Methacrylsäure-Copolymer, Hyaluronsäure, Myristinsäure, Palmitinsäure, Stearinsäure, Behensäure, Cellulose oder Derivate davon, Maltose, Dextran, Glucomannan, Glucosamin, Chitosan, Heparin, Alginat, Inulin, Stärke, Glycogen, Chitin, Chondroitin, Dextrin, Keratansulfat, Rindertalg, Walwachs, Bienenwachs, Paraffinwachs und Castorwachs.

## Revendications

1. Corps intégré à micro-aiguilles électriques, comprenant :
une électrode d'électroporation qui doit être mise en contact avec la peau d'un corps humain pour appliquer une impulsion de champ électrique, incluant une partie de base, une pluralité de parties d'électrode qui ressortent de la partie de base, et un jeu de micro-aiguilles,
une micro-aiguille colée à chaque partie d'électrode et pouvant être insérée dans la peau d'un corps humain, chaque micro-aiguille comprenant un matériau biocompatible et biodégradable ainsi qu'un matériau génétique,
dans lequel chaque micro-aiguille est destinée à se dégrader dans la peau, et une impulsion de champ électrique doit être appliquée via l'électrode pour une électroporation dans un site où les micro-aiguilles doivent être insérées pour permettre une introduction en douceur du matériau génétique contenu dans les micro-aiguilles dans des cellules,
moyennant quoi les micro-aiguilles peuvent être préparées en mélangeant un matériau visqueux biocompatible et biodégradable et le matériau génétique dans une composition mélangée,
**caractérisé en ce que** les micro-aiguilles peuvent être formées en fournissant la composition mélangée à l'état liquide dans chaque partie de la pluralité de parties d'électrode qui ressortent de la partie de base et en faisant coaguler la composition mélangée.

2. Corps intégré à micro-aiguilles électriques selon la revendication 1, dans lequel le matériau visqueux biocompatible et biodégradable est sélectionné parmi le groupe constitué par les composés polyester, polyhydroxyalcanoate (PHA), poly(acide α-hydroxylé), poly(acide β-hydroxylé), poly(3-hydroxybutyrate-co-valérate) (PHBV), poly(3-hydroxyproprionate) (PHP), poly(3-hydroxyhexanoate) (PHH), poly(4-hydroxyacide), poly(4-hydroxybutyrate), poly(4-hydroxyvalérate), poly(4-hydroxyhexanoate), polyester amide, polycaprolactone, acide polylactique (PLA), acide polyglycolique (PGA), acide poly(lactique-co-glycolique) (PLGA), polydioxanone, poly-orthoester, polyanhydride, acide polyglycolique-co-carbonate de triméthylène, poly-phosphoester, poly-phosphoester uréthane, acide polyaminé, polycyanoacrylate, polycarbonate de triméthylène, carbonate de polyimine, carbonate de polytyrosine, polycarbonate, arylate de polytyrosine, oxalate de polyalkylène, polyphosphagène, PHA-PEG, polyvinylpyrrolidone, polybutadiène, acide polyhydroxybutyrique, poly(méthacrylate de méthyle), ester de l'acide polyméthacrylique, polypropylène, polystyrène, poly(diéthylaminoacétate d'acétale de vinyle), poly(acétate de vinyle), poly(alcool de vinyle), poly(butyral de vinyle), poly(formal de vinyle), copolymère chlorure de vinyle-propylène-acétate de vinyle, copolymère chlorure de vinyle-acétate de vinyle, polymère cumarone-indène, dibutylamino hydroxypropyl éther, copolymère éthylène-acétate de vinyle, distéarate de glycérol, copolymère 2-méthyl-5-vinylpyridine méthacrylate-acide méthacrylique, acide hyaluronique, acide myristique, acide palmitique, acide stéarique, acide béhénique, cellulose ou ses dérivés, maltose, dextrane, glucomannane, glucosamine, chitosane, héparine, alginate, inuline, amidon, glycogène, chitine, chondroïtine, dextrine, sulfate de kératane, suif de boeuf, cire de baleine, cire d'abeille, paraffine et cire de castor.

3. Procédé de préparation d'un corps intégré à micro-aiguilles électriques, comprenant :
dans une première étape, la préparation d'une électrode d'électroporation incluant une partie de base et une pluralité de parties d'électrode qui ressortent de la partie de base ;
dans une deuxième étape, la préparation d'une composition mélangée en mélangeant un matériau visqueux biocompatible et biodégradable et un matériau génétique ; et
dans une troisième étape, la formation d'une micro-aiguille sur chaque partie d'électrode en alimentant la composition mélangée à l'état liquide sur chacune des parties d'électrode et en faisant coaguler la composition mélangée.

4. Procédé selon la revendication 3, dans lequel la formation de la micro-aiguille comprend :
l'application de la composition mélangée à l'état liquide sur un substrat en forme de plaque ;
la mise en contact des parties d'électrode avec la composition mélangée appliquée ;
l'étirement de la composition mélangée en réalisant un mouvement relatif des parties d'électrode au-dessus du substrat ; et
la coagulation de la composition mélangée étirée.

5. Procédé selon la revendication 4, comprenant en outre, après la coagulation de la composition mélangée étirée, la séparation de la composition mélangée coagulée du substrat en liquéfiant la composition mélangée sur un côté du substrat en chauffant le substrat.

6. Procédé selon la revendication 3, dans lequel la formation de la micro-aiguille comprend :
le mouchetage de la composition mélangée à l'état liquide sur les parties d'électrode ; et
la coagulation de la composition mélangée mouchetée.

7. Procédé selon la revendication 3, dans lequel le matériau visqueux biocompatible et biodégradable est sélectionné parmi le groupe constitué par les composés polyester, polyhydroxyalcanoate (PHA), poly(acide α-hydroxylé), poly(acide β-hydroxylé), poly(3-hydroxybutyrate-co-valérate) (PHBV), poly(3-hydroxyproprionate) (PHP), poly(3-hydroxyhexanoate) (PHH), poly(4-hydroxyacide), poly(4-hydroxybutyrate), poly(4-hydroxyvalérate), poly(4-hydroxyhexanoate), polyester amide, polycaprolactone, acide polylactique (PLA), acide polyglycolique (PGA), acide poly(lactique-co-glycolique) (PLGA), polydioxanone, poly-orthoester, polyanhydride, acide polyglycolique-co-carbonate de triméthylène, poly-phosphoester, poly-phosphoester uréthane, acide polyaminé, polycyanoacrylate, polycarbonate de triméthylène, carbonate de polyimine, carbonate de polytyrosine, polycarbonate, arylate de polytyrosine, oxalate de polyalkylène, polyphosphagène, PHA-PEG, polyvinylpyrrolidone, polybutadiène, acide polyhydroxybutyrique, poly(méthacrylate de méthyle), ester de l'acide polyméthacrylique, polypropylène, polystyrène, poly(diéthylaminoacétate d'acétale de vinyle), poly(acétate de vinyle), poly(alcool de vinyle), poly(butyral de vinyle), poly(formal de vinyle), copolymère chlorure de vinyle-propylène-acétate de vinyle, copolymère chlorure de vinyle-acétate de vinyle, polymère cumarone-indène, dibutylamino hydroxypropyl éther, copolymère éthylène-acétate de vinyle, distéarate de glycérol, copolymère 2-méthyl-5-vinylpyridine méthacrylate-acide méthacrylique, acide hyaluronique, acide myristique, acide palmitique, acide stéarique, acide béhénique, cellulose ou ses dérivés, maltose, dextrane, glucomannane, glucosamine, chitosane, héparine, alginate, inuline, amidon, glycogène, chitine, chondroïtine, dextrine, sulfate de kératane, suif de boeuf, cire de baleine, cire d'abeille, paraffine et cire de castor.
